# DEMANDE DE BREVET EUROPEEN

(11) **EP 4 755 390 A1**
(43) Date de publication de la demande: **10.06.2026**
(21) Numéro de dépôt: 25220251.0
(22) Date de dépôt: 02.12.2025
(51) Int. Cl.: A61K 36/9068, A61P 29/00, A61K 9/14, A61K 36/28, A61K 9/00, A61K 9/16

(54) **COMPOSITION COMPRENANT DE L'ACHILLÉE ET DU GINGEMBRE DESTINÉE À UNE ADMINISTRATION PAR VOIE ORALE CHEZ UN SUJET, POUR SON UTILISATION DANS LA RÉDUCTION ET/OU LA PRÉVENTION DE LA DOULEUR**

(30) Priorité: 03.12.2024 FR 2413366
(71) Demandeur: Larena, 49270 Oree D'Anjou (FR)
(72) Inventeur: LEBLANC, Anne, 49110 Montrevault Sur Evre (FR); DUBOURDEAUX, Michel, 03140 Taxat (FR)
(74) Mandataire: Jacobacci & Partners France

(57) **Abrégé**

La présente invention concerne une composition destinée à une administration par voie orale chez un sujet, pour son utilisation dans la réduction et/ou la prévention de la douleur, notamment de douleurs abdominopelviennes, notamment de dysménorrhées.

La composition comprend une combinaison de préparations de plantes :
- une poudre d'Achillée millefeuille,
- une poudre de gingembre, et
- un extrait concentré d'Achillée millefeuille.

## Description

### Domaine technique de l'invention

La présente invention concerne le domaine technique des compositions destinées à une administration par voie orale chez un sujet, pour son utilisation dans la réduction et/ou la prévention de la douleur, notamment de douleurs abdominopelviennes, notamment de dysménorrhées (dites encore « règles douloureuses »).

### Etat de la technique

La réduction et/ou la prévention de la douleur, qu'elle soit aiguë ou chronique, est un objectif fondamental en médecine.

Différentes approches thérapeutiques sont utilisées pour soulager la douleur, notamment les médicaments analgésiques, les anti-inflammatoires, les thérapies physiques et les approches psychologiques.

Cependant, de nombreux patients continuent de souffrir de douleurs insuffisamment contrôlées, avec un impact significatif sur leur qualité de vie.

La recherche de nouvelles solutions thérapeutiques et préventives efficaces et bien tolérées pour la prise en charge de la douleur reste donc un enjeu majeur de santé publique.

A cet égard, la douleur abdominopelvienne, et notamment la dysménorrhée, représente un fardeau considérable pour un grand nombre de femmes, et ce, dès l'adolescence.

Des études récentes ont mis en lumière l'ampleur et l'impact de ce problème :
- la dysménorrhée est extrêmement fréquente : près de 90% des femmes en âge de procréer sont concernées, et 50% à 90% des adolescentes ;
- la douleur peut être intense : 42% des femmes souffrant de dysménorrhée la décrivent comme intense ;
- la dysménorrhée a un impact significatif sur la vie des femmes : elle est la principale cause d'absentéisme scolaire chez les adolescentes et peut entraîner une restriction des activités quotidiennes ;
- la dysménorrhée est souvent banalisée et sous-traitée : 66% des femmes considèrent la douleur menstruelle comme normale et 61 % n'ont jamais consulté pour ce motif.

L'état de la technique met en évidence un besoin crucial de nouvelles solutions thérapeutiques et préventives pour la prise en charge de la douleur.

Ces solutions doivent être :
- efficaces : capables de soulager significativement la douleur, y compris les douleurs intenses ;
- bien tolérées : avec un minimum d'effets secondaires, pour une utilisation à long terme si nécessaire ;
- faciles à administrer : pour favoriser l'observance, notamment chez les jeunes filles ;
- adaptées aux besoins spécifiques des femmes : en tenant compte de l'âge, de l'intensité de la douleur et des autres traitements éventuels.

### Présentation de l'invention

Afin de remédier à l'inconvénient précité de l'état de la technique, la présente invention propose une nouvelle composition pour administration orale destinée à la réduction et/ou la prévention de la douleur, notamment de douleurs abdominopelviennes, et plus particulièrement de dysménorrhées.

Cette composition comprend une combinaison spécifique de préparations de plantes, à savoir :
- une poudre d'Achillée millefeuille,
- une poudre de gingembre, et
- un extrait concentré d'Achillée millefeuille.

De manière générale, la composition selon l'invention présente divers avantages :
- elle est efficace pour réduire la douleur abdominopelvienne, notamment les dysménorrhées ;
- elle est bien tolérée et ne présente pas d'effets secondaires indésirables ;
- elle est facile à administrer par voie orale ;
- elle est formulée à partir d'ingrédients naturels et respectueux de l'environnement.

Sans être limité par une quelconque théorie, cette action biologique est assurée par une action synergique sur l'inhibition de la COX-2 et de la 5-LOX, deux enzymes impliquées dans l'inflammation et la douleur.

D'autres caractéristiques non limitatives et avantageuses du produit conforme à l'invention, prises individuellement ou selon toutes les combinaisons techniquement possibles, sont les suivantes :
- ladite combinaison de préparations de plantes est sous forme d'une poudre imprégnée dans laquelle ledit extrait concentré d'Achillée millefeuille est imprégné et séché sur ladite poudre d'Achillée millefeuille et/ou ladite poudre de gingembre ;
- ladite combinaison de préparations de plantes est sous forme d'une poudre imprégnée présentant les caractéristiques granulométriques suivantes : une taille inférieure, ou égale, à 50 µm représentant au maximum 25%, en poids, une taille inférieure, ou égale, à 100 µm représentant au maximum 65%, en poids, et une taille inférieure, ou égale, à 500 µm représentant au minimum 80%, en poids ; de préférence, ladite poudre imprégnée présente une masse volumique apparente allant de 0,3 à 0,8 g/cm3, et une masse volumique tassée allant de 0,4 à 0,9 g/cm3 ;
- ledit extrait concentré d'Achillée millefeuille consiste en un extrait aqueux et/ou hydro-alcoolique ;
- ladite poudre de gingembre est issue de racines de gingembre ;
- ladite composition provoque une diminution de l'activité COX-2 / 5-LOX ;
- ladite composition se présente sous la forme de comprimés.

La présente invention concerne encore un procédé de fabrication, pour la fabrication d'une composition selon l'invention, comprenant :
- une étape d'imprégnation, au cours de laquelle ledit extrait d'Achillée millefeuille est imprégné sur ladite poudre de gingembre et/ou ladite poudre d'Achillée millefeuille, et
- une étape de séchage, au cours de laquelle ladite au moins une poudre imprégnée est séchée.

Selon un mode de réalisation particulier, ledit procédé de fabrication comprend une étape de micronisation de ladite poudre d'Achillée millefeuille et de ladite poudre de gingembre, dont les paramètres sont ajustés pour obtenir une poudre imprégnée présentant, après ladite étape de séchage, les caractéristiques granulométriques suivantes :
- une taille inférieure, ou égale, à 50 µm représentant au maximum 25%, en poids,
- une taille inférieure, ou égale, à 100 µm représentant au maximum 65%, en poids, et
- une taille inférieure, ou égale, à 500µm représentant au minimum 80%, en poids. La présente invention concerne encore une utilisation d'une composition destinée à une administration par voie orale chez un sujet, pour son utilisation dans la réduction et/ou la prévention de la douleur, notamment de douleurs abdominopelviennes, notamment de dysménorrhées,
laquelle composition comprend une combinaison de préparations de plantes :
- une poudre d'Achillée millefeuille,
- une poudre de gingembre, et
- un extrait concentré d'Achillée millefeuille.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée qui illustrent des formes, non limitatives, de réalisation de l'invention.

La présente invention concerne ainsi une composition destinée à une administration par voie orale chez un sujet, pour son utilisation dans la réduction et/ou la prévention de la douleur, notamment de douleurs abdominopelviennes, notamment de dysménorrhées (dites encore règles douloureuses).

Par « composition », on entend avantageusement un mélange ou une association de différents constituants, notamment des principes actifs issus de préparations de plantes, et éventuellement des excipients, formulé pour l'administration du produit.

Par « voie orale », on entend avantageusement une voie d'administration d'une composition qui implique l'ingestion de la composition par la bouche, permettant ainsi son absorption par le système digestif.

Par « sujets », on entend en particulier un sujet humain souffrant de douleurs, en particulier de douleurs chroniques ou aiguës.

Les sujets humains peuvent également comprendre des personnes en bonne santé désirant réduire ou prévenir des douleurs, notamment les femmes ou adolescentes cherchant à prévenir ou réduire les douleurs abdominopelviennes, telles que les dysménorrhées.

Le terme « réduire la douleur » se réfère à une utilisation pour réduire, soulager ou éliminer la douleur.

Le terme « prévention de la douleur » se réfère à une utilisation pour prévenir ou réduire la douleur avant qu'elle ne se produise.

De manière générale, et selon l'invention, cette composition comprend pour cela une combinaison de préparations de plantes :
- une poudre d'Achillée millefeuille,
- une poudre de gingembre, et
- un extrait concentré d'Achillée millefeuille.

De préférence, la présente composition comprend les préparations suivantes, exprimés en pourcentage massique par rapport à la masse totale de la composition :
- un extrait et/ou une poudre d'Achillée millefeuille représentant entre 40 et 50 % du mélange total, et
- une poudre de gingembre présent à hauteur de 50 à 60 %.

De préférence, le ratio d'extraction pour l'Achillée millefeuille est d'au minimum 2,2 à 3 pour 1. Concernant le gingembre, le ratio d'extraction est d'au minimum 0,5 à 0,8 pour 1, de préférence.

De manière générale, par « préparation de plante », on englobe avantageusement les préparations obtenues à partir des matières premières végétales, notamment en les réduisant en poudre et/ou en les traitant par un procédé d'extraction, de distillation, d'expression, de fractionnement, de purification, de concentration ou de fermentation.

### Préparation d'Achillée millefeuille

L'Achillée millefeuille, connue scientifiquement sous le nom *d'Achillea millefolium* L., est une plante herbacée vivace appartenant à la famille des Astéracées.

Tel qu'abordé ci-dessus, la composition selon l'invention comprend deux préparations différentes d'Achillée millefeuille :
- une poudre d'Achillée millefeuille,
- un extrait concentré d'Achillée millefeuille.

De préférence, la poudre d'Achillée millefeuille et l'extrait concentré d'Achillée millefeuille sont issus des sommités fleuries.

De préférence, les « sommités fleuries » désignent les parties supérieures d'une plante, comprenant les fleurs et les tiges associées.

Par « extrait concentré d'Achillée », on entend avantageusement une fraction obtenue par extraction à partir d'une matière végétale puis par concentration de ladite fraction extraite.

La concentration des composés d'intérêt qui sont présents dans l'extrait végétal concentré est supérieure à la concentration des composés d'intérêt qui sont présents dans la matière végétale d'origine.

A cet effet, l'extrait végétal concentré est issu d'une opération d'extraction à partir d'une matière végétale suivie d'une opération de concentration dudit extrait végétal.

De telles opérations d'extraction / concentration sont par exemple décrites dans le document EP-2 080 436 ou dans le document EP-4 159 198.

Une telle opération d'extraction comprend avantageusement les étapes suivantes :
- une étape de contact entre la matière végétale d'intérêt et un solvant,
- au moins une étape d'extraction, proprement dite, pour l'extraction d'au moins une partie des principes actifs présents dans ladite matière végétale d'intérêt (par migration dans le solvant).

L'opération de concentration consiste alors avantageusement à concentrer les principes actifs par élimination d'au moins une partie du solvant d'extraction.

La concentration est avantageusement d'un facteur de concentration d'au moins 5, de préférence d'un facteur de concentration de 8 à 15.

De préférence, l'extrait concentré d'Achillée millefeuille consiste en un extrait aqueux et/ou hydro-alcoolique (de préférence un extrait aqueux).

Plus généralement, différents procédés d'extraction sont adaptés à l'obtention de l'extrait concentré d'Achillée millefeuille selon l'invention.

Selon un mode de réalisation, le procédé d'extraction consiste en un procédé de préparation d'une préparation végétale, à partir de matière végétale, comprenant :
- une étape de contact entre la matière végétale et un solvant,
- au moins une étape d'extraction en phase liquide des principes actifs, réalisée sous chauffage et sous pression réduite, pour obtenir une fraction d'extraction contenant lesdits principes actifs et une matière végétale résiduelle, et
- une étape de séchage de ladite matière végétale résiduelle en présence de ladite fraction d'extraction, afin de permettre la fixation des principes actifs sur ladite matière végétale résiduelle.

Un tel procédé est par exemple décrit encore dans le document EP-2 080 436.

### Préparation de gingembre

La poudre de gingembre est avantageusement issue de racines de gingembre.

Par « gingembre », on entend avantageusement le *Zingiber officinale* Roscoe, une plante herbacée vivace de la famille des Zingibéracées.

Par « racine de gingembre », on entend avantageusement le rhizome du gingembre, c'est-à-dire la tige souterraine horizontale de la plante, qui est la partie utilisée en médecine traditionnelle et en alimentation.

### Poudre imprégnée

Selon un mode de réalisation préféré, la combinaison de préparations de plantes est sous forme d'une poudre imprégnée dans laquelle l'extrait concentré d'Achillée millefeuille est imprégné et séché sur la poudre d'Achillée millefeuille et/ou la poudre de gingembre.

En d'autres termes, l'extrait concentré d'Achillée millefeuille est imprégné et séché sur :
- la poudre d'Achillée millefeuille, ou
- la poudre de gingembre, ou
- le mélange de la poudre d'Achillée millefeuille et de la poudre de gingembre.

Encore de manière générale, la poudre imprégnée selon l'invention comprend avantageusement :
- une poudre de gingembre,
- une préparation d'Achillée millefeuille, sous forme d'une poudre et sous forme d'un extrait concentré.

Par « imprégné et séché », on entend avantageusement un procédé dans lequel l'extrait concentré d'Achillée millefeuille est déposé sur la poudre d'Achillée millefeuille et/ou la poudre de gingembre de manière à pénétrer la structure poreuse de la poudre, puis séché afin d'éliminer le solvant et d'obtenir une poudre imprégnée stable et homogène.

L'imprégnation peut être réalisée par différents procédés, tels que la pulvérisation, le malaxage ou l'enrobage.

Le séchage peut être effectué par différents moyens, tels que le séchage par air chaud, le séchage sous vide ou la lyophilisation.

Le choix du procédé d'imprégnation et de séchage dépendra notamment de la nature de l'extrait concentré, des caractéristiques des poudres et des propriétés souhaitées pour la poudre imprégnée finale.

Toujours de manière générale, la combinaison de préparations de plantes est avantageusement sous forme d'une poudre imprégnée présentant les caractéristiques granulométriques suivantes :
- une taille inférieure, ou égale, à 50 µm représentant au maximum 25%, en poids,
- une taille inférieure, ou égale, à 100 µm représentant au maximum 65%, en poids, et
- une taille inférieure, ou égale, à 500µm représentant au minimum 80%, en poids.

De manière classique en soi, les caractéristiques granulométriques sont avantageusement issues d'une technique granulométrique par tamisage.

Le pourcentage en masse en question représente avantageusement la partie de la poudre imprégnée qui reste sur le tamis, dit encore « refus » ou « refus par tamis ».

De telles valeurs peuvent être mesurées selon un protocole décrit par exemple dans le document suivant : Pharmacopée Européenne 9.0, « 2.9.38. Distribution granulométrique par tamisage analytique » ou Pharmacopée Européenne 9.0, « 2.9.35. Finesse des poudres ».

De préférence encore, la poudre imprégnée présente :
- une masse volumique apparente allant de 0,3 à 0,8 g/cm3, et
- une masse volumique tassée allant de 0,4 à 0,9 g/cm3.

La masse volumique apparente et la masse volumique tassée sont avantageusement obtenues par le procédé décrit dans le document Pharmacopée Européenne 9.7, « 2.9.34. Masse volumique vrac et masse volumique après tassement ».

Les caractéristiques d'une poudre imprégnée sont par exemple décrites dans le document EP-4 159 198.

De manière générale, selon l'invention, l'étape de micronisation est avantageusement mise en œuvre sur le support végétal :
- préalablement à l'étape d'imprégnation, de sorte à ajuster les caractéristiques granulométriques du support végétal avant l'étape d'imprégnation, et/ou
- entre l'étape d'imprégnation et l'étape de séchage, de sorte à ajuster les caractéristiques granulométriques du support végétal imprégné non-séché, et/ou
- postérieurement à l'étape de séchage, de sorte à ajuster les caractéristiques granulométriques du support végétal imprégné et séché.

De manière générale, un procédé de fabrication d'une poudre imprégnée, ainsi que les paramètres caractéristiques d'une poudre, sont décrites par exemple dans le document EP-4 159 198.

De préférence, la posologie est de 1200 à 1500 mg de la préparation de plante répartis en 1 à 5 prises par jour (idéalement, une prise toutes les 8 heures environ). Ce schéma de traitement est avantageusement suivi pendant au moins les 2 à 3 premiers jours des menstruations, ou pendant les 1 à 5 cinq jours complets des menstruations, et ce, sur une durée minimale de 2 à 5 cycles consécutifs.

De préférence, la posologie quotidienne comprend 500 à 1000 mg de préparation de gingembre et 500 à 1000 mg de préparation d'Achillée, avec un ratio de 4 à 6:1 pour la combinaison poudre d'Achillée millefeuille / extrait concentré d'Achillée millefeuille. Cette composition équivaut à 2500 à 3500 mg en équivalent plante par jour.

### Composition phyto-chimique

Selon un mode de réalisation, la composition selon l'invention comprend avantageusement les molécules suivantes (sur la base d'une analyse LC-MS) : glucose, saccharose, acide citrique, acide chlorogénique, acide caféique, lutéoline-7-o-glucoside, zingiberoside C, lutéoline-4-o-glucoside + apigénine-7-o-glucoside, C20H24O6, lutéoline, apigénine, C23H28O8, 6-gingerdoine, 6-gingerol.

De préférence, la composition comprend les terpènes suivants : 6-shogaol, 10-gingerol, 8-gingerol et 6-gingerol.

### Diminution de l'activité COX-2 / 5-LOX

Selon un mode de réalisation préféré, la composition provoque une diminution de l'activité COX-2 / 5-LOX.

De manière générale, l'activité COX-2 / 5-LOX peut être mesurée en utilisant des kits d'essai enzymatique spécifiques, tels que ceux commercialisés par Cayman Chemical.

De manière générale, pour l'activité COX-2, l'essai implique par exemple la conversion de l'acide arachidonique en PGF2, dont la production est quantifiée par une réaction couplée produisant un signal mesurable (colorimétrique ou fluorimétrique). L'ajout de composés inhibiteurs potentiels permet d'évaluer leur effet sur la diminution de la production de PGF2, révélant ainsi leur capacité à inhiber l'enzyme et leur potentiel anti-inflammatoire.

Pour l'activité 5-LOX, l'essai mesure de préférence l'inhibition de l'enzyme 5-lipoxygénase en quantifiant la production de peroxydes lipidiques formés lors de la conversion de l'acide arachidonique en leucotriènes. Cette méthode repose sur une détection colorimétrique qui permet d'évaluer l'efficacité des inhibiteurs dans la réduction de l'activité 5-LOX.

La diminution d'activité est de préférence d'au moins 30%, sans être limitatif.

Ces deux enzymes, la cyclooxygénase-2 (COX-2) et la 5-lipoxygénase (5-LOX), jouent un rôle clé dans la cascade inflammatoire et la production de médiateurs de la douleur, notamment les prostaglandines et les leucotriènes.

En inhibant l'activité de la COX-2 et de la 5-LOX, la composition selon l'invention permet de réduire la production de ces médiateurs inflammatoires et de soulager efficacement la douleur, notamment la douleur abdominopelvienne et les dysménorrhées.

Tel que démontré dans les exemples, l'association des différents composants de la composition, à savoir la poudre d'Achillée millefeuille, la poudre de gingembre et l'extrait concentré d'Achillée millefeuille, permet d'obtenir une synergie d'action sur l'inhibition de la COX-2 et de la 5-LOX.

### Galénique

Dans un mode de réalisation avantageux et non limitatif, la composition selon l'invention se présente sous la forme de comprimés, pelliculés ou non, de préférence à avaler.

Par « comprimé », on entend avantageusement la forme galénique solide obtenue par compression d'une poudre ou d'un granulé contenant une ou plusieurs substances actives et des excipients.

Cette forme galénique offre plusieurs avantages pour l'administration de la composition : facilité d'administration, protection des principes actifs, amélioration de la stabilité et libération contrôlée.

Les comprimés peuvent être obtenus par des techniques de fabrication standard, en utilisant des excipients couramment utilisés dans l'industrie pharmaceutique ou non-pharmaceutique, tels que des agents liants, des diluants, des lubrifiants et des agents de pelliculage.

Plus généralement, la composition selon l'invention contient avantageusement une combinaison d'ingrédients adaptés à une utilisation dans l'industrie pharmaceutique, cosmétologique, agro-alimentaire, de la nutrition (y compris les denrées alimentaires destinées à des fins médicales spéciales - DADFMS), des compléments alimentaires, de la nutrition animale.

La composition selon l'invention comprend ainsi avantageusement :
- une composition selon l'invention, pour son utilisation chez des sujets humains, adapté à un traitement et/ou la prévention de la douleur, et
- au moins un support ou un excipient, acceptable en fonction du l'utilisation envisagée, par exemple de la pulpe de fruit de baobab et/ou de la maltodextrine.

La composition de la présente invention peut être préparé sous la forme d'une composition, qui peut être une formulation telle que des comprimés, des gélules, des capsules, des poudres, des granules, des solutions, des pastilles, des gelées, des préparations de crème, des sirops, des suspensions, des teintures, des aérosols et autres.

La composition de la présente invention peut également être préparée sous la forme d'une composition non-pharmaceutique.

La composition non-pharmaceutique englobe les compositions cosmétologiques, les compositions agro-alimentaires, les compositions nutritionnelles, les compléments alimentaires.

La composition non-pharmaceutique peut être préparée par des techniques de préparation généralement connues, et des additifs acceptables peuvent être ajoutés à la composition non-pharmaceutique.

Par exemple, la composition non-pharmaceutique selon l'invention consiste avantageusement en un complément alimentaire.

Par « complément alimentaire », on entend avantageusement les compléments alimentaires qui sont soumis à l'ensemble des dispositions générales du droit alimentaire mais aussi aux règles spécifiques définies par la directive 2002/46/CE du Parlement européen et du Conseil du 10 juin 2002 relative au rapprochement des législations des États membres concernant les compléments alimentaires, transposée en droit français par le décret n°2006-352.

Par « compléments alimentaires », on englobe avantageusement les denrées alimentaires dont le but est de compléter le régime alimentaire normal et qui constituent une source concentrée de nutriments ou d'autres substances ayant un effet nutritionnel ou physiologique seuls ou combinés, commercialisés sous forme de doses, à savoir les formes de présentation telles que les ampoules de liquide, les flacons munis d'un compte-gouttes et les autres formes analogues de préparations liquides.

De manière générale, la composition peut encore contenir souche probiotique, qui englobe en particulier les microorganismes vivants qui, lorsqu'ils sont ingérés en quantité suffisante, exercent des effets positifs sur la santé, au-delà des effets nutritionnels traditionnels.

La souche probiotique convenant à l'invention est physiologiquement acceptable. En d'autres termes, cette souche probiotique peut être administrée sans risques à l'animal ou l'homme.

Les souches probiotiques englobent notamment les genres lactobacilles (bactéries du genre *Lactobacillus*), bifidobactéries (bactéries du genre *Bifidobacterium*), streptocoques (bactéries du genre *Streptococcus*) ou lactocoques.

Encore de manière générale, des exemples spécifiques de microorganismes probiotiques sont *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus, Lactobacillus amylovorus, Lactobacillus casei (Shirota), Lactobacillus rhamnosus (souche GG), Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonii, Lactobacillus paracasei, Lactobacillus plantarum, Lactobacillus reuteri, Lactobacillus salivarius, Lactobacillus alimentarius, Lactobacillus curvatus, Lactobacillus casei subsp. casei, Lactobacillus sake Lactococcus lactis, Enterococcus (faecalis, faecium), Lactococcus lactis (subspp lactis ou cremoris), Leuconstoc mesenteroides subsp dextranicum, Pediococcus acidilactici, Sporolactobacillus inulinus, Streptococcus salvarius subsp. Thermophilus, Streptococcus thermophilus, Staphylococccus carnosus, Staphylococcus xylosus, Saccharomyces (cerevisiae ou* encore *boulardii), Bacillus (cereus var toyo ou subtilis), Bacillus coagulans, Bacillus licheniformis, Escherichia coli strain nissle, Propionibacterium freudenreichii* et leurs mélanges.

Plus particulièrement, il s'agit de microorganismes probiotiques issus du groupe des bactéries lactiques, comme notamment les *Lactobacillus* et/ou les *Bifidobacterium.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei ou Bifidobacterium bifidum, Bifidobacterium breve, Bifidobacterium longum, Bifidobacterium animalis, Bifidobacterium lactis, Bifidobacterium infantis, Bifidobacterium adolescentis ou Bifidobacterium pseudocatenulatum* et leurs mélanges.

De manière générale, ladite au moins une souche probiotique est avantageusement choisie parmi les souches probiotiques vivantes ou les souches probiotiques inactivées.

Par forme « vivante », on entend une forme dotée de la capacité à se multiplier sous réserve de la placer dans un environnement propice à la récupération de cette capacité.

Ainsi, au sens de la présente invention, le terme vivant couvre l'état dit dormance dans lequel les microorganismes peuvent être placés suite à un traitement physicochimique tel que par exemple la lyophilisation.

De manière générale, la souche probiotique est avantageusement mise en œuvre sous une forme lyophilisée.

Ce type de formulation possède les avantages d'être facilement accessible, de mise en œuvre aisée et de ne soulever aucune difficulté et/ou contrainte en termes de stockage. Par ailleurs, il est compatible avec le conditionnement de microorganismes à l'état de dormance.

Cette lyophilisation peut être réalisée selon des méthodes conventionnelles ou selon une méthode décrite par exemple dans le document FR-3 103 827.

Par ailleurs, le terme « inactivé » désigne des souches ayant subi un traitement visant à les tuer. De tels traitements peuvent consister, à titre d'exemple non limitatif, en un traitement en autoclave, par ultra-sons, une homogénéisation haute pression ou encore un choc osmotique.

Les souches inactivées sont avantageusement intactes.

La souche probiotique est avantageusement inactivée par la chaleur, dite encore « souche probiotique tyndallisée ».

La composition peut également contenir des postbiotiques, correspondent à des préparations de micro-organismes, avantageusement choisis parmi les souches probiotiques précitées, inanimés et/ou de leurs composants conférant un bénéfice pour la santé de l'hôte.

La composition peut encore contenir d'autres types d'ingrédients, par exemple des vitamines, des minéraux ou tout autre ingrédient nutritionnel.

Plus particulièrement, la composition peut contenir des ingrédients nutraceutiques comme la vitamine C, la glucosamine, la chondroïtine, le palmitoyléthanolamide, la vitamine B6 ou des acides gras.

### Utilisation non-thérapeutique

La présente invention englobe encore une utilisation non-thérapeutique d'une composition selon l'invention, pour son utilisation chez des sujets humains, dans le traitement et/ou la prévention de la douleur.

Une telle utilisation non-thérapeutique s'étend ainsi à l'utilisation de cette composition chez des sujets humains en dehors d'un contexte thérapeutique, c'est-à-dire indépendamment du traitement d'une pathologie.

La composition selon l'invention est en effet adaptée à prévenir l'apparition de douleurs liées à diverses situations telles que des événements physiologiques (règles douloureuses, etc.).

Ainsi, la composition selon l'invention peut être utilisée à titre préventif par des sujets sains souhaitant anticiper et limiter l'apparition de douleurs dans ces situations.

Par exemple, des femmes sujettes aux dysménorrhées pourraient prendre la composition, avant leurs règles, pour atténuer les douleurs éventuelles.

Cette utilisation non-thérapeutique offre une alternative naturelle et préventive aux traitements classiques, permettant de réduire la consommation de médicaments et d'améliorer le bien-être général des individus.

### Réduction et/ou prévention de la douleur

Par « douleur », on entend notamment les douleurs abdominopelviennes, notamment de dysménorrhées.

Les douleurs abdominopelviennes sont des douleurs localisées dans la région du bassin et de l'abdomen, qui peuvent avoir diverses origines, notamment gynécologiques, digestives, urologiques ou musculo-squelettiques.

Les dysménorrhées, quant à elles, sont des douleurs menstruelles qui surviennent avant ou pendant les règles. Elles sont souvent décrites comme des crampes ou des douleurs spasmodiques dans le bas-ventre, et peuvent s'accompagner d'autres symptômes tels que des nausées, des vomissements, des maux de tête ou de la fatigue.

De manière générale, par « réduction et/ou prévention de la douleur », la présente invention englobe une diminution de son intensité et/ou de sa durée.

Par « intensité » de la douleur, on englobe la douleur mesurée par des outils pour la caractériser et l'évaluer.

De manière générale, des questionnaires et des échelles de douleur permettent d'en décrire les manifestations, et d'en mesurer l'intensité ainsi que l'impact sur la qualité de vie.

Pour les adultes, l'échelle la plus utilisée est l'échelle numérique ou l'échelle visuelle analogique, graduée de 0 pour une absence de douleur, à 10 pour la douleur maximale imaginable.

De manière générale, la présente invention englobe une diminution de l'intensité et/ou de la durée pour la douleur mesurée chez un sujet recevant la composition selon l'invention par rapport à l'intensité et/ou à la durée pour la douleur mesurée chez un sujet ne recevant pas la composition selon l'invention.

La douleur englobe en particulier :
- les douleurs légères,
- les douleurs modérées, et
- les douleurs sévères.

Les douleurs légères sont des douleurs peu intenses qui peuvent être facilement supportées par le sujet et n'affectent pas ou peu son activité quotidienne.

Les douleurs modérées sont plus intenses et peuvent affecter le quotidien du sujet, en limitant par exemple ses mouvements ou sa capacité à travailler.

Les douleurs sévères sont les douleurs les plus intenses, qui peuvent être invalidantes et empêcher le sujet de mener une vie normale.

### Procédé de fabrication

L'invention concerne également un procédé de fabrication pour la composition décrite précédemment.

Ce procédé comprend les étapes suivantes :
- une étape d'imprégnation

Au cours de cette étape, l'extrait concentré d'Achillée millefeuille est imprégné sur la poudre de gingembre et/ou la poudre d'Achillée millefeuille. L'imprégnation peut être réalisée par différents procédés, tels que la pulvérisation, le malaxage ou l'enrobage, de manière à obtenir une distribution homogène de l'extrait sur la poudre. Ce procédé permet notamment de favoriser la stabilité et la biodisponibilité des principes actifs dans la composition finale.
- une étape de séchage

Après l'imprégnation, la poudre imprégnée est séchée afin d'éliminer le solvant (eau et/ou alcool) utilisé pour l'extraction. Le séchage peut être effectué par différents moyens, tels que le séchage par air chaud, le séchage sous vide ou la lyophilisation. Les paramètres de séchage sont optimisés pour préserver l'intégrité des principes actifs et obtenir une poudre sèche et stable.

Selon un mode de réalisation préféré, le procédé de fabrication comprend une étape de micronisation de ladite poudre d'Achillée millefeuille et de ladite poudre de gingembre, dont les paramètres sont ajustés pour obtenir une poudre imprégnée présentant, après ladite étape de séchage, les caractéristiques granulométriques suivantes :
- une taille inférieure, ou égale, à 50 µm représentant au maximum 25%, en poids,
- une taille inférieure, ou égale, à 100 µm représentant au maximum 65%, en poids, et
- une taille inférieure, ou égale, à 500µm représentant au minimum 80%, en poids.

Par « micronisation », on entend avantageusement l'opération ayant pour objet la réduction du support végétal en particules ayant des dimensions de l'ordre du micromètre (ou micron).

Le paramétrage de l'étape de micronisation, classique en soi, est notamment fonction de la technologie utilisée (par exemple au moyen d'un microniseur ou d'un broyeur) et de l'ordre des étapes du procédé de fabrication.

Une telle étape de micronisation est par exemple décrite dans le document général suivant : Powder Technology Handbook, Fourth Edition, Higashitani et al. Il est encore possible de se référer au document EP-4 159 198.

Bien entendu, diverses autres modifications peuvent être apportées à l'invention dans le cadre des revendications annexées.

### Exemples

### Exemple 1 : Mise en évidence in tubo de l'effet synergique de la composition sur l'inhibition des activités COX-2 et 5-LOX

Différents modes de préparations des deux plantes, et différentes façons de les associer, ont été testés.

Nous avons pu montrer un effet synergique sur l'inhibition des enzymes COX-2 et 5-LOX avec ces deux plantes, lorsqu'elles sont préparées conjointement.

### Préparations testées

- Extrait aqueux Achillée 4:1
- Extrait aqueux Achillée 4:1 séché sur poudre de gingembre + poudre d'achillée
- Poudre de gingembre
- Extrait gingembre (extrait 3.5 :1)
- Extrait aqueux Achillée 4:1 + extrait de gingembre 3,5 :1.

### Approche expérimentale

Une approche expérimentale *in tubo* basée sur la mesure de l'activité de la cyclooxygénase-2 (COX-2) et de l'activité de la 5-lipooxygénase (5-LOX), en absence et présence des différents éléments d'essai, a été proposée.

L'activité COX de l'enzyme a été évaluée par la mesure du taux de prostaglandine PGF2a formée après incubation de l'enzyme avec l'acide arachidonique.

L'activité LOX de l'enzyme a été évaluée par la mesure du taux d'hydropéroxydes formés après incubation de l'enzyme avec l'acide linoléique.

### Résultats

Les résultats sont illustrés au travers des tableaux ci-dessous, respectivement, pour l'inhibition de la cyclooxygénase-2 (COX-2) en Tableau 1 et de la 5-lipooxygénase (5-LOX) en Tableau 2.

**[Tableau 1]**

| | %Inhibition COX-2 |
|---|---|
| Extrait Achillée (0,649 µg/ml) | 9 |
| Poudre de gingembre (1,1 µg/ml) | -2 |
| Extrait de gingembre 3,5:1 (0,185 µg/ml) | 4 |
| Extrait Achillée (0,649 µg/ml) + Extrait de gingembre 3,5:1 (0,185 µg/ml) | 27 |
| Extrait aqueux Achillée séché sur poudre de gingembre + poudre d'achillée (1,749 µg/mL) | 57 |

**[Tableau 2]**

| | %Inhibition 5-LOX |
|---|---|
| Extrait Achillée (1298 µg/ml) | 2 |
| Poudre de gingembre (2200 µg/ml) | 43 |
| Extrait de gingembre 3,5:1 (370 µg/ml) | -10 |
| Extrait Achillée (1298 µg/ml) + Extrait de gingembre 3,5:1 (370 µg/ml) | 8 |
| Extrait aqueux Achillée séché sur poudre de gingembre + poudre d'achillée (3498 µg/ml) | 60 |

Les résultats illustrent que, à quantité égale d'extrait d'achillée, l'association avec le gingembre est plus avantageuse dans la préparation *extrait aqueux Achillée 4:1 séché sur poudre de gingembre + poudre d'achillée,* pour atteindre des valeurs d'inhibition intéressantes des activités COX-2 et 5-LOX (inhibition d'au moins 30%).

Ce mode de préparation fait apparaitre un effet synergique entre les préparations de plantes.

### Conclusion

Ces données démontrent que le fait d'associer des plantes entre elles permet de développer une préparation de plantes avec action combinée COX-2/5-LOX.

Le mode de préparation de ces plantes peut révéler une action synergique entre les plantes mises en jeu.

Une telle composition, destinée à une administration par voie orale chez un sujet, serait ainsi adaptée à une utilisation dans la réduction et/ou la prévention de la douleur, notamment de douleurs abdominopelviennes, notamment de dysménorrhées.

### Exemple 2 : Evaluation de l'efficacité et de la tolérance de la composition chez le sujet humain (Etude en vie réelle)

Afin de confirmer que les résultats obtenus *in tubo* sur l'inhibition des voies COX-2 et 5-LOX se traduisent en bénéfice clinique, une étude en conditions réelles d'utilisation a été menée sur une cohorte de sujets humains féminins, afin d'évaluer l'efficacité de la composition selon l'invention sur les douleurs dysménorrhéiques.

### 1. Protocole de l'étude

L'étude a été réalisée sur une population de 50 volontaires sains de sexe féminin, âgées de 12 à 35 ans (moyenne d'âge : 23 ans).

L'étude s'est déroulée sur 3 cycles menstruels :
- T0 (Cycle de référence) : Observation des symptômes sans supplémentation ;
- T1 et T2 : Cycles avec administration de la composition par voie orale.

La posologie appliquée était de 3 comprimés par jour, pendant la durée des règles, correspondant à la posologie.

La composition administrée comprenait la combinaison de poudre de gingembre, de poudre d'Achillée millefeuille et d'extrait concentré d'Achillée millefeuille.

Plus précisément, les ingrédients sont : Poudre de gingembre (*Zingiber officinale Roscoe,* rhizome), extrait d'achillée millefeuille (extrait et poudre d'*Achillea millefolium L.,* sommité fleurie), pulpe du fruit du baobab (*Adansonia digitata L.*), maltodextrine, antiagglomérant : acides gras ; vitamine B6.

La préparation de plantes est sous forme d'une poudre imprégnée présentant les caractéristiques granulométriques suivantes :
- une taille inférieure, ou égale, à 50 µm représentant au maximum 25%, en poids,
- une taille inférieure, ou égale, à 100 µm représentant au maximum 65%, en poids, et
- une taille inférieure, ou égale, à 500µm représentant au minimum 80%, en poids.

La poudre imprégnée présente :
- une masse volumique apparente allant de 0,3 à 0,8 g/cm3, et
- une masse volumique tassée allant de 0,4 à 0,9 g/cm3.

L'évaluation a été réalisée via des auto-questionnaires standardisés incluant des Échelles Visuelles Analogiques (EVA) de 1 à 10 pour mesurer l'intensité de la douleur.

### 2. Résultats sur l'intensité de la douleur (Scores EVA)

Les résultats démontrent une diminution significative de l'intensité des douleurs dès le premier cycle de supplémentation (T1), effet qui se renforce au deuxième cycle (T2).

L'évolution des scores moyens de douleur (EVA sur 10) entre le cycle témoin (T0) et après deux cycles de traitement (T2) est résumée ci-dessous :
- concernant les douleurs générales liées aux règles, le score moyen a diminué de 7,56 à T0 pour atteindre 6,06 à T2, ce qui correspond à une réduction de 20 %.
- s'agissant des douleurs localisées dans le bas-ventre, le score moyen est passé de 7,58 à T0 à 6,02 à T2, représentant une diminution de 21 %.
- pour les crampes abdominales, une réduction de 24 % a été observée, le score moyen passant de 7,50 à T0 à 5,63 à T2.

Enfin, pour les douleurs situées au niveau des reins (douleurs lombaires), le score moyen a diminué de 7,13 à T0 à 5,54 à T2, soit une baisse de 21 %.

On observe notamment que :
- 70 % des sujets constatent une diminution de l'intensité des douleurs générales liées aux règles ;
- 86 % des sujets constatent une diminution de l'intensité des crampes abdominales ;
- la charge symptomatique moyenne (nombre de problèmes simultanés) a diminué, passant de 8,94 à T0 à 5,8 en fin de supplémentation.

### 3. Rapidité d'action

Parmi les sujets ayant ressenti une diminution des inconforts, un premier effet a été ressenti en moyenne en 35 minutes, et un résultat significatif sur les douleurs est observé après environ 2 jours de prise. Cela confirme l'adéquation de la composition pour une prise en charge rapide de la douleur aiguë.

### 4. Qualité de vie et Tolérance

L'efficacité antalgique s'accompagne d'une amélioration globale du bien-être :
80 % des sujets constatent une amélioration de leur qualité de vie pendant leurs règles dès la fin du premier cycle ;
72 % des sujets rapportent une diminution de l'absentéisme (scolaire ou professionnel) ;

Enfin, concernant la sécurité d'usage, la composition a présenté une excellente tolérance : 100 % des volontaires n'ont rapporté aucun effet indésirable au cours de l'étude.

### Conclusion

Ces résultats cliniques confirment la synergie d'action mise en évidence dans l'Exemple 1. La composition selon l'invention permet une réduction significative, rapide et bien tolérée des douleurs abdominopelviennes et des dysménorrhées chez la femme.

## Revendications

1. Composition destinée à une administration par voie orale chez un sujet, pour son utilisation dans la réduction et/ou la prévention de la douleur, notamment de douleurs abdominopelviennes, notamment de dysménorrhées,
laquelle composition comprend une combinaison de préparations de plantes :
- une poudre d'Achillée millefeuille,
- une poudre de gingembre, et
- un extrait concentré d'Achillée millefeuille.

2. Composition pour son utilisation selon la revendication 1, dans laquelle ladite combinaison de préparations de plantes est sous forme d'une poudre imprégnée dans laquelle ledit extrait concentré d'Achillée millefeuille est imprégné et séché sur ladite poudre d'Achillée millefeuille et/ou ladite poudre de gingembre.

3. Composition pour son utilisation selon l'une quelconque des revendications 1 ou 2, dans laquelle ladite combinaison de préparations de plantes est sous forme d'une poudre imprégnée présentant les caractéristiques granulométriques suivantes :
- une taille inférieure, ou égale, à 50 µm représentant au maximum 25%, en poids,
- une taille inférieure, ou égale, à 100 µm représentant au maximum 65%, en poids, et
- une taille inférieure, ou égale, à 500µm représentant au minimum 80%, en poids.

4. Composition pour son utilisation selon la revendication 3, dans laquelle ladite poudre imprégnée présente :
- une masse volumique apparente allant de 0,3 à 0,8 g/cm3, et
- une masse volumique tassée allant de 0,4 à 0,9 g/cm3.

5. Composition pour son utilisation selon l'une quelconque des revendications 1 à 4, dans laquelle ladite poudre d'Achillée millefeuille et ledit extrait concentré d'Achillée millefeuille sont issus des sommités fleuries.

6. Composition pour son utilisation selon l'une quelconque des revendications 1 à 5, dans laquelle ledit extrait concentré d'Achillée millefeuille consiste en un extrait aqueux et/ou hydro-alcoolique.

7. Composition pour son utilisation selon l'une quelconque des revendications 1 à 6, dans laquelle ladite poudre de gingembre est issue de racines de gingembre.

8. Procédé de fabrication, pour la fabrication d'une composition selon l'une quelconque des revendications 1 à 7, comprenant :
- une étape d'imprégnation, au cours de laquelle ledit extrait d'Achillée millefeuille est imprégné sur ladite poudre de gingembre et/ou ladite poudre d'Achillée millefeuille, et
- une étape de séchage, au cours de laquelle ladite au moins une poudre imprégnée est séchée.

9. Procédé selon la revendication 8, **caractérisé en ce que** ledit procédé de fabrication comprend une étape de micronisation de ladite poudre d'Achillée millefeuille et de ladite poudre de gingembre, dont les paramètres sont ajustés pour obtenir une poudre imprégnée présentant, après ladite étape de séchage, les caractéristiques granulométriques suivantes :
- une taille inférieure, ou égale, à 50 µm représentant au maximum 25%, en poids,
- une taille inférieure, ou égale, à 100 µm représentant au maximum 65%, en poids, et
- une taille inférieure, ou égale, à 500µm représentant au minimum 80%, en poids.

10. Utilisation d'une composition destinée à une administration par voie orale chez un sujet, pour son utilisation dans la réduction et/ou la prévention de la douleur, notamment de douleurs abdominopelviennes, notamment de dysménorrhées,
laquelle composition comprend une combinaison de préparations de plantes :
- une poudre d'Achillée millefeuille,
- une poudre de gingembre, et
- un extrait concentré d'Achillée millefeuille.
